# EUROPEAN PATENT SPECIFICATION

(11) **EP 0 545 941 B1**
(45) Date of publication and mention of the grant of the patent: **15.11.1995**
(21) Application number: 91912023.8
(22) Date of filing: 05.06.1991
(51) Int. Cl.: H04R 1/10

(54) **AN EAR PROTECTOR**
OHRSCHUTZVORRICHTUNG
DISPOSITIF DE PROTECTION POUR LES OREILLES

(30) Priority: 18.06.1990 SE 9002170
(43) Date of publication of application: 16.06.1993
(73) Proprietor: ROSEN, Gösta, 161 51 Bromma (SE)
(72) Inventor: ROSEN, Gösta, 161 51 Bromma (SE)
(74) Representative: Lindblom, Erik J.
(86) International application number: SE9100399
(87) International publication number: WO9120161

(56) References cited:
- EP-A- 0 389 174
- DE-A- 3 607 885
- US-A- 3 952 158

## Description

### TECHNICAL FIELD

The present invention relates to an ear protector which has the features stated in the preamble of claim 1.

### BACKGROUND ART

Ear protectors of the aforesaid kind augmented with a telephone receiver are known to the art and are used normally when performing work in noisy environments, in which the noise levels exceed 85dB(A), and where there is a need for the workmen present to communicate with one another through speech signals.

It is also known that in order to converse in such noisy environments, it is necessary for such ear protectors to function as telephone devices, i.e. the protectors must be provided with a microphone and telephone receiver, the latter in the form of a receiving capsule.

It is also known to install in the earmuff of the ear protector a plate onto which the telephone receiver can be secured and to enclose the plate and the telephone receiver in a pourous sound damping material.

Also known to the art are several different cordless or wireless information transmission systems, adapted for use with modulated photodiodes, so-called IR-light or IR-beams, where light is transmitted by special IR-photodiodes and is received by special diodes, so-called PIN-diodes.

An example of the earlier known art is found in equipment intended for single-path remote control of television sets and video tape recorders. Other single-path applications have also been proposed in the art.

Particularly significant to the present invention is a two-path information transmission system, in which modulated IR-light or IR-beams are used for speech communication between people present in a restricted space or between a person present within a restricted space and a person present outside said space.

This enables fully acceptable speech communication to be carried out between persons who are in close proximity of one another in a noisy environment and between these persons and persons located outside said noisy environment.

A system which affords the possibility of such communication is described in more detail in the U.S. Patent Specification No. 4,648,131, in which the requisite photodiodes are mounted on a helmet.

Swedish Patent Application SE-A-8903572, filed on the 26th October, 1989 and published on the 27th April, 1991, illustrates and describes a two-path cordless information transmission system in which IR-beam generating diodes and IR-beam receiving diodes, so-called PIN-diodes, are mounted on the earmuffs of the ear protector.

It can be mentioned that it is known that certain plastic materials tend to exhibit good transparent properties for IR-light but have the tendency of strongly damping permeability for light of other wavelengths. Finally an car protector, with the features stated in the preamble of claim 1, is known through the publication DE-A- 36 07 883.

### DISCLOSURE OF THE PRESENT INVENTION

### TECHNICAL PROBLEMS

When considering the earlier state of the art, as described above, it will be seen that a technical problem resides in the provision of means which render it no longer necessary to mount separate photodiodes on an earmuff or earmuffs of an ear protector, and subsequent to forming a hole in the earmuff or earmuffs, to enable these diodes to be connected to electronic circuits mounted within the earmuff or earmuffs, while still presenting a simple construction.

It will also be seen that a further technical problem resides in positioning the requisite light-transmitting and light-receiving diodes intended for speech communication with an ear protector in a manner such as to eliminate the use of a separate guard, on either the earmuffs or the helmet as protection against mechanical damage from an external source.

In this respect, a qualified technical problem is one of realizing that the aforesaid technical problems can be solved by placing all diodes within respective earmuffs and by positioning or otherwise arranging said diodes within said earmuffs or earmuff in a manner such that the light-transmitting diodes are able to transmit IR-light without being affected by the light-receiving diodes which receive IR-light.

Another technical problem is one of configuring the earmuff or earmuffs in a particular manner and producing said earmuff or earmuffs from a particular material such as to enable the diodes to be mounted within the earmuff/earmuffs of the ear protector, said material not only being transparent to infrared light but also capable of being moulded.

It will also be seen that a further technical problem resides in the selection of a plastic material which has properties that will enable the earmuffs to be extrusion-moulded and/or cast in moulds, and which also exhibits a colour and/or other properties which render respective earmuffs transparent to infrared light, with only small losses.

It will also be seen that a further technical problem resides in the provision of conditions, with the aid of simple means, such that one or more diodes that are intended to receive modulated IR-beams can be mounted at a small distance from or towards the inner surface of a respective earmuff, without the damping engendered by the material from which the earmuff is made and the colour selected reaching unacceptable values.

It will also be seen that a further technical problem is one of selecting from all available plastic materials a plastic material which is suitable for said purpose and to realize that the plastic material selected shall have properties equal to or at least substantially corresponding to the properties of the plastic material known as "IR-MAKROLON"®.

Another technical problem is one of realizing the importance of mounting one or more IR-light transmitting diodes adjacent the inner, concave surface of the earmuff.

It will also be seen from the known prior art described above that a technical problem resides in realizing the importance of mounting the requisite diodes and requisite electronic circuits and telephone receiver on a printed circuit board and also to realize the advantages that are afforded when the IR-light transmitting diodes are mounted on the actual circuit board itself while the IR-receiving diodes are mounted close to the inner surface of the earmuff and in spaced relationship with the transmitting diodes.

It will also be seen that a technical problem resides in enabling the earmuffs to be produced in the same injection moulding tools or casting tools as those earlier used to produce conventional earmuffs, despite the addition of diodes and electronic circuits to said earmuffs, and thereby save tool costs.

### SOLUTION

The present invention provides a solution to one or more of the aforesaid technical problems and is based on an ear protector with the features mentioned in the preamble of claim 1.

In accordance with the invention an ear protector has the features mentioned in claim 1.

Further embodiments of the invention are defined in the subclaims.

### ADVANTAGES

The advantages primarily afforded by an ear protector according to the invention lie in the possibility of achieving the transmission of information, particularly the transmission of speech signals, with the aid of modulated IR-beams, by using a circuit board on which or to which transmitting and receiving diodes are mounted or connected, and which is placed within the earmuff. By producing the earmuff from a plastic material which is pervious to IR-beams, there is provided an ear protector which requires the provision of no additional devices on the external surfaces of the earmuff of said protector or in a recess formed therein to this end, while the requisite diodes are well protected against mechanical damage.

### BRIEF DESCRIPTION OF THE DRAWINGS

An embodiment of an inventive ear protector, at present preferred, will now be described in more detail with reference to the accompanying drawings, in which
- Figure 1: is a perspective view, partly in section,. of an ear protector having two earmuffs, each of which has enclosed therein a respective printed circuit board having mounted thereon a number of IR-beam transmitting diodes and a number of IR-beam receiving diodes;
- Figure 2: is a top view illustrating the principle of a printed circuit board significant to the invention; and
- Figure 3: is a sectional view of a receiving telephone according to Figure 1.

### DESCRIPTION OF EMBODIMENTS AT PRESENT PREFERRED

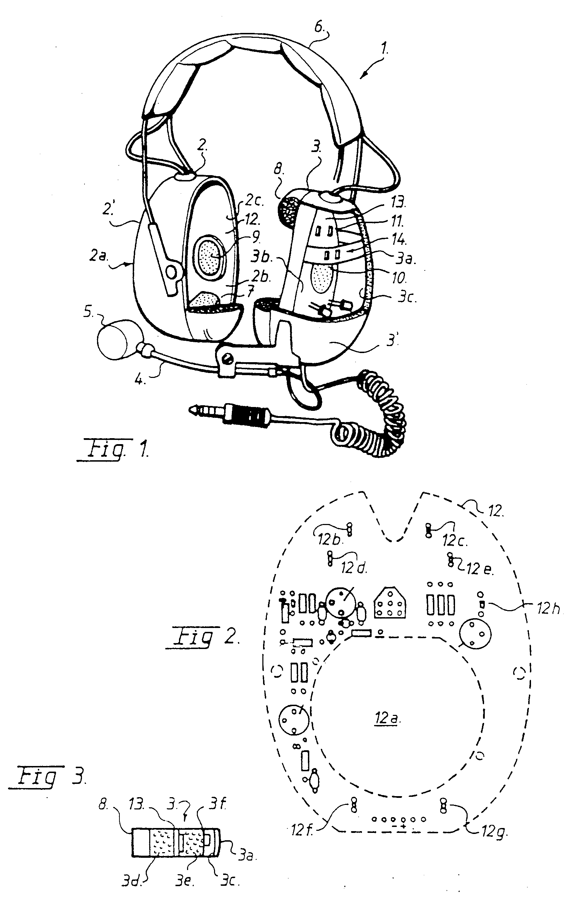

Figure 1 illustrates in perspective an ear protector 1 comprising two sound-attenuating earmuffs 2, 3 and a headstrap 6 carrying the earmuffs.

Parts of the earmuffs have been removed, for the sake of illustration.

The earmuffs 2 and 3 are principly identical to one another, with the exception that a microphone bar 4 with associated microphone 5 is mounted on the frame or body 3′ of one earmuff, and that the necessary electrical connection passes through the earmuff to a circuit enclosed therein.

Each earmuff 2, 3 has formed therein an opening which is dimensioned to extend over a respective ear of the person wearing the ear protector.

The earmuffs 2, 3 are carried by an extensible head-strap 6, to which respective earmuff frames or bodies 2′ and 3′ are pivotally connected.

Each earmuff 2, 3 and earmuff frame 2′, 3′ has an outer, convex surface 2a, 3a and, in the case of the illustrated embodiment, is provided with means 2b, 3b (not shown in detail) which hold a ring 7, 8, which is intended to surround a respective ear and lie against the head of the wearer.

Each earmuff 2 and 3 has enclosed therein a telephone receiver 9 and 10. At least one of the earmuffs is equipped with one such telephone receiver, so as to enable information signals received in the form of modulated infrared radiation, or IR-beams, to be reproduced with the aid of said receiver telephone and the requisite electronic circuits.

The circuitry and the conditions required for transmitting and receiving information with the aid of IR-beams is known to the art.

There is nothing to prevent the recommendations illustrated and described in the aforesaid Swedish patent application from being used with the illustrated embodiment, and hence these components and the manner in which the system works will not be described in detail here.

For the sake of illustration, certain parts of the earmuff illustrated in Figure 1 have been removed so as to show the positioning of the circuit board 12, 13 used.

According to the present invention, the earmuff frame 2′, 3′ is made from a plastic material which is transparent to IR-radiation, and one or more IR-radiation receiving PIN-diodes 14 are mounted in spaced relationship with the circuit board, on the inner surface 2c, 3c of the earmuff and the earmuff frame.

The earmuff frame will preferably be made of an extrudable and/or mouldable plastic, whose properties are equal to, or at least substantially comparable with, the properties of the plastic material retailed under the trademark IR-MAKROLON®.

One or more IR-light transmitting diodes 11 is/are mounted on or adjacent to the printed circuit board 13.

The diodes 14 intended for receiving IR-beams are connected electrically to the circuit board 12 in the earmuff 2 and to the circuit board 13 in the earmuff 3, whereas the diodes 11, intended for transmitting IR-beams, are fixedly or permanently mounted on said circuit boards.

The respective telephone receivers 9 and 10 are held by said circuit boards 12 and 13 in a known manner, and respective circuit boards are positioned centrally adjacent (at an adapted distance from) the apertured part of the earmuff, which faces towards the surrounding ring 7 or 8.

Figure 2 is a top view of a printed circuit board 12.

The requisite electronic components are only shown schematically, although it shall be noted that the telephone receiver 9 is accommodated in a centrally positioned aperture 12a.

A number of IR-beam transmitting diodes are fixedly connected to the circuit board by means of respective connections 12b-12g. These connections are thus made directly to the circuit board.

Connected in parallel with the connection 12h are a number of IR-beam receiving diodes 14, so-called PIN-diodes. These diodes are placed side-by-side on a holder strip and are intended to lie close to the inner surface of a respective earmuff, preferably with the arrangement of a sound-insulating layer between the PIN-diodes and the circuit board 13.

Figure 3 is a sectional view of the earmuff 3. Figure 3 illustrates the ring 8, a sound-damping foam-rubber layer or like layer 3d, the circuit board 13 with the necessary components and the six IR-beam transmitting diodes 11, a sound-attenuating, foam-rubber layer or like layer 3e and a strip 3f, on which the requisite PIN-diodes 14 are mounted in side-by-side relationship.

It will be understood that the invention is not restricted to the described and illustrated exemplifying embodiment thereof and that modifications can be made within the scope of the invention as defined in the following Claims.

It is to be noted that the material sold under the Trade Mark MAKROLON®, by Bayer AG, Leverkusen, Germany, shall preferably have the colour 45/401. The expression IR-MACROLON® is used to indicate that the material is transparent for IR radiation.

## Claims

1. An ear protector comprising two earmuffs (2, 3) which cover the ears of the person wearing the protector, a headstrap (6) or the like which mutually connect the earmuffs and an earmuff frame (2′, 3′), wherein respective earmuff frames (2′, 3′) have an outer convex surface and are preferably provided with means for securing a ring (7, 8) which encircles a respective ear of the wearer, and further comprising a telephone receiver (9, 10) enclosed in at least one earmuff and operative to reproduce information received via IR-beams, **characterized** in that the earmuff frame (2′, 3′) comprises a plastic material which is transparent at least to IR-radiation; and in that one or more IR-radiation receiving diodes (14) is/are mounted on or adjacent the inner surface of the earmuff (2, 3) and earmuff frame (2′, 3′).

2. An ear protector according to Claim 1,
**characterized** in that the earmuff frame (2′, 3′) is made of an extrudable and/or mouldable plastic material whose properties are equal to or at least correspond to the properties of the material "IR-MAKROLON"®.

3. An ear protector according to Claim 1 or 2, **characterized** in that one or more IR-radiation transmitting diodes (11) is/are mounted adjacent the inner, concave surface of the earmuff (2, 3) or earmuff frame (2′, 3′).

4. An ear protector according to Claim 1, 2 or 3, **characterized** in that the IR-radiation receiving diodes (14) are mounted on a printed circuit board (12, 13), whereas the IR-radiation transmitting diodes (11) are mounted on or close to said circuit board (12, 13).

5. An ear protector according to Claim 1 or 4, **characterized** in that the telephone receiver (9, 10) is mounted centrally and held firmly by said circuit board (12, 13).

6. An ear protector according to Claim 1, 4 or 5, **characterized** in that the circuit board (12, 13) is placed adjacent to and at a distance from that part of the earmuff (2, 3) which faces towards the surrounding ring (7, 8).

## Patentansprüche

1. Ohrschutzvorrichtung mit zwei Ohrschützern (2, 3), die die Ohren der die Schutzvorrichtung tragenden Person bedecken, einem Kopfbügel (6) oder dergleichen, der die Ohrschützer miteinander verbindet, und einem Ohrschützerrahmen (2′, 3′), wobei die entsprechenden Ohrschützerrahmen (2′, 3′) eine konvexe Außenfläche aufweisen und vorzugsweise mit Mitteln zur Sicherung eines Ringes (7, 8) versehen sind, der ein entsprechendes Ohr des Trägers umgibt, und weiterhin mit einem in mindestens einem Ohrschützer eingeschlossenen Fernsprechempfänger (9, 10) zur Wiedergabe von über Infrarotstrahlen empfangenen Informationen, dadurch gekennzeichnet, daß der Ohrschützerrahmen (2′, 3′) einen mindestens für Infrarotstrahlung transparenten Kunststoff umfaßt, und daß eine oder mehrere Infrarotstrahlung empfangende Diode(n) (14) auf oder neben der Innenfläche des Ohrschützers (2, 3) und Ohrschützerrahmens (2′, 3′) befestigt ist/sind.

2. Ohrschutzvorrichtung nach Anspruch 1, dadurch gekennzeichnet, daß der Ohrschützerrahmen (2′, 3′) aus spritzbarem und/oder verformbarem Kunststoff hergestellt ist, dessen Eigenschaften den Eigenschaften des Materials "IR-MAKROLON"® gleich sind oder diesen mindestens entsprechen.

3. Ohrschutzvorrichtung nach Anspruch 1 oder 2, dadurch gekennzeichnet, daß neben der konkaven Innenfläche des Ohrschützers (2, 3) oder Ohrschützerrahmens (2′, 3′) eine oder mehrere Infrarotstrahlung übertragende(n) Diode(n) (11) befestigt ist/sind.

4. Ohrschutzvorrichtung nach Anspruch 1, 2 oder 3, dadurch gekennzeichnet, daß die Infrarotstrahlung empfangenden Dioden (14) auf einer Leiterplatte (12, 13) befestigt sind, während die Infrarotstrahlung übertragenden Dioden (11) auf oder in der Nähe der besagten Leiterplatte (12, 13) befestigt sind.

5. Ohrschutzvorrichtung nach Anspruch 1 oder 4, dadurch gekennzeichnet, daß der Fernsprechempfänger (9, 10) mittig befestigt und von der besagten Leiterplatte (12, 13) festgehalten ist.

6. Ohrschutzvorrichtung nach Anspruch 1, 4 oder 5, dadurch gekennzeichnet, daß die Leiterplatte (12, 13) neben dem dem umgebenden Ring (7, 8) zugekehrten Teil des Ohrschützers (2, 3) plaziert und von diesem Teil beabstandet ist.

## Revendications

1. Dispositif de protection pour les oreilles comprenant deux coquilles (2,3) qui couvrent les oreilles de la personne portant le dispositif de protection, une sangle (6) ou pièce assimilée qui relient [sic] mutuellement les coquilles et une monture de coquille (2′,3′), dans lequel les montures de coquille (2′,3′) respectives ont une surface convexe externe et sont de préférence dotées d'un moyen pour fixer un anneau (7,8) qui encercle une oreille respective de la personne portant le dispositif, et comprenant en outre un récepteur téléphonique (9,10) enfermé dans au moins une coquille et fonctionnant pour reproduire des informations reçues par l'intermédiaire de rayons infrarouges, caractérisé en ce que la monture de coquille (2′,3′) comprend une matière plastique qui est transparente au moins au rayonnement infrarouge; et en ce qu'une ou plusieurs diodes réceptrices de rayonnement infrarouge (14) est/sont montée(s) sur ou près de la surface interne de la coquille (2,3) et de la monture de coquille (2′,3′).

2. Dispositif de protection pour les oreilles selon la revendication 1, caractérisé en ce que la monture de coquille (2′,3′) est faite en une matière plastique pouvant être extrudée et/ou moulée dont les propriétés sont égales aux propriétés de la matière "IR-MAKROLON"® ou qui au moins y correspondent.

3. Dispositif de protection pour les oreilles selon la revendication 1 ou 2, caractérisé en ce qu'une ou plusieurs diodes émettrices de rayonnement infrarouge (11) est/sont montée(s) près de la surface concave, interne de la coquille (2,3) ou de la monture de coquille (2′,3′).

4. Dispositif de protection pour les oreilles selon la revendication 1, 2 ou 3, caractérisé en ce que les diodes réceptrices de rayonnement infrarouge (14) sont montées sur une carte de circuit imprimé (12,13) tandis que les diodes émettrices de rayonnement infrarouge (11) sont montées sur ou près de ladite carte de circuit (12,13)

5. Dispositif de protection pour les oreilles selon la revendication 1 ou 4, caractérisé en ce que le récepteur téléphonique (9,10) est monté centralement et maintenu fermement par ladite carte de circuit (12,13).

6. Dispositif de protection pour les oreilles selon la revendication 1, 4 ou 5, caractérisé en ce que la carte de circuit (12,13) est placée près et à une certaine distance de la partie de la coquille (2,3) qui fait face à l'anneau entourant (7,8).
